Europäisches Patentamt

European Patent Office

Office européen des brevets

(19)

(11) Veröffentlichungsnummer : **0 065 316 B2**

(12) ## NEUE EUROPÄISCHE PATENTSCHRIFT

(45) Veröffentlichungstag der neuen Patentschrift :
**08.01.92 Patentblatt 92/02**

(51) Int. Cl.⁵ : **A47C 27/00, A47C 23/00**

(21) Anmeldenummer : **82104371.8**

(22) Anmeldetag : **18.05.82**

(54) **Betteil.**

(30) Priorität : **20.05.81 DE 3120081**

(43) Veröffentlichungstag der Anmeldung :
**24.11.82 Patentblatt 82/47**

(45) Bekanntmachung des Hinweises auf die
Patenterteilung :
**12.12.84 Patentblatt 84/50**

(45) Bekanntmachung des Hinweises auf die
Entscheidung über den Einspruch :
**08.01.92 Patentblatt 92/02**

(84) Benannte Vertragsstaaten :
**AT BE CH DE FR GB IT LI NL**

(56) Entgegenhaltungen :
**DE-A- 2 259 001**

(56) Entgegenhaltungen :
**DE-A- 2 916 110**
**DE-U- 7 401 791**
**FR-A- 2 187 985**
**FR-A- 2 436 590**
**FR-A- 2 455 870**
**US-A- 3 818 521**

(73) Patentinhaber : **Matherm GmbH**
**Wiesenstrasse 18**
**W-6433 Philippsthal (DE)**

(72) Erfinder : **Matherm GmbH**
**Wiesenstrasse 18**
**W-6433 Philippsthal (DE)**

(74) Vertreter : **Geyer, Ulrich F., Dr., Dipl.Phys.**
**Wagner & Geyer Patentanwälte Postfach 22 14**
**39 Gewürzmühlstrasse 5**
**W-8000 München 22 (DE)**

EP 0 065 316 B2

Jouve, 18, rue Saint-Denis, 75001 PARIS

## Beschreibung

Die Erfindung betrifft ein Betteil, insbesondere ein Ober- oder Unterbett, das eine Einlage bzw. eine Gewebeeinlage mit Kupferelementen aufweist.

Aus der FR-A-2 436 590 ist ein Matratzenschoner bekannt, bei dem auf einem Tuch oder Textilgewebe Kupferelemente in Form von Plaketten oder Kabelstücken zum Schutz gegen kosmische und von Wasseradern herrührenden Strahlen vorgesehen sind. Diese Kupferelemente sind nicht gleichmäßig über die gesamte Fläche des Matratzenschoners und darüber hinaus auch nicht ohne Unterbrechungen verteilt. Darüber hinaus können sich diese Kupferplaketten bzw. Kupfer-Kabelstücke leicht von der Textilgewebe Auflage lösen, da sich der Schläfer im Bett wälzt und bewegt und daher die Verbindung zwischen den Kupferelementen und der Unterlage leicht gelöst wird.

Aus der FR-A-2 455 870 ist ein Matratzenschoner bekannt, bei dem ein Gewebe aus Kupferfäden parallel und ohne Verbindung zu einem anderen Textilgewebe angeordnet ist. Das Baumwollgewebe und das davon unabhängige Kupfergewebe können sich auf Grund der Bewegung des Schläfers zueinander leicht verschieben, so daß neben der Tatsache, daß keine Verbindung zwischen den Kupferelementen und der Gewebeeinlage besteht, auch eine gleichmäßige Verteilung der Kupferelemente über die Matratzenschonerfläche hinweg nicht gewährleistet ist.

Aus der DE-A-2 259 001 ist ein Wasserbett-Heizgerät bekannt, bei dem das Heizdrahtsystem aus Endlos-Kupferlitzen hergestellt ist. Diese Kupferlitzen sind in einer besonderen, sich nicht überlappenden Anordnung vorgesehen. Sie weisen insbesondere keine Maschen- oder Gewebeausbildung auf, da sonst ein Kurzschluß der stromführenden Litzen auftreten würde.

Wenn zwei verschiedene Stoffe z.B. durch Reitung mitteinander in Berührung kommen, so weisen die Berührungsflächen nach der Trennung eine Ladung auf. An der Berührungsfläche herrscht ein starkes elektrisches Feld, in dem die Moleküle polarisiert werden. Der höheren Dielektrizitätskonstante entspricht eine stärkere Verschiebung der Ladungen innerhalb der Moleküle. Sie verlieren leicht Elektronen an den anderen Stoff und bleiben daher aufgeladen zurück. Dieses Phänomen wird in der Physik unter dem Begriff Berührungs- und Reibungselektrizität zusammengefaßt. Stoffe, die bei Berührung oder Reibung elektrostatisch aufgeladen werden, sind insbesonder auch Baumwolle, Seide, Federn, Kunststoff usw. Der Grad der Aufladung hängt sehr wesentlich von der Oberfläche und Veränderung von derselben, z.B. von adsorbierten Gasen, anhaftender Flüssigkeit usw. ab. Bei Betten, sowohl bei Unterbetten als auch insbesondere bei Oberbetten führt die Berührungs- und Reibungselektrizität aufgrund der ständigen Berührung und Reibung zwischen den einzelnen Materialen, aus denen das Bett besteht, z.B. zwischen Baumwolle und Seide, Baumwolle und Federn, Seide und Federn usw. zu erheblichen Aufladungen, die insbesondere durch körperliche Ausdünstungen während des Schlafs relativ hohe Werte erreichen. In Zusammenhang mit der vorliegenden Erfindung wurden statische Aufladungen von 20 000 Volt und darüber in Betten gemessen und sind keine Seltenheit.

Elektrostatische Felder beeinträchtigen jedoch das Schlafverhalten und das Wohlgefühl des Menschen physiologisch erheblich. Die Person schläft weniger tief und weniger lang und fühlt sich auch in einem solchen Bett weniger wohl.

Der Erfindung, liegt die Aufgabe zugrunde, ein Betteil, insbesondere ein Ober- oder Unterbett zu schaffen, bei dem elektrostatische Felder im wesentlichen nicht auftreten bzw. vermieden werden, so daß der Schläfer besser, tiefer und länger schlafen kann und sich besser fühlt.

Diese Aufgabe wird erfindungsgemäß dadurch gelöst, daß die Kupferelemente in Form von Kupferlitzen in die Gewebeeinlage eingewebt sind.

Die gestellte Aufgabe wird auch dadurch gelöst, daß die Kupferelemente aus in Form von auf die Gewebeeinlage aufgedampftem Kupfer besteht. Durch diese erfindungsgemäßen Merkmale bzw. durch die erfindungsgemäße Ausgestaltung der Kupferelemente wird eine im wesentlichen gleichmäßige Ausbildung der Kupferelemente über die Gewebeeinlage erreicht und die Kupferelemente sind fest und dauerhaft mit der Gewebeeinlage verbunden.

Gemäß einer weiteren alternativen Form der Erfindung wird die gestellte Aufgabe bei einem Betteil, das eine Einlage mit Kupferelementen aufweist, auch dadurch gelöst, daß die Einfage eine mit Kupfer bedampfte Folie ist. Auch dadurch ergibt sich eine gleichmäßige Verteilung bzw. Ausgestaltung der Kupferelemente uber die Einlage hinweg, so daß auch dadurch die allgemeine erfinderische Idee realisiert ist.

Durch die erfindungsgemäßen Merkmale, die Kupferelemente in Form von Kupferlitzen in die Gewebeeinlage einzuweben bzw. auf die Gewebeeinlage aufzudampfen, ergibt sich eine gleichmäßige Verteilung der Kupferelemente über das Betteil hinweg sowie eine innige Verbindung der Kupferelemente mit der Gewebeeinlage. Dadurch wird neben einer hohen mechanischen Stabilität erreicht, daß sich eine gutleitende, gleichmäßige Fläche ergibt und sich keine bzw nur sehr geringe elektrostatische Felder aufbauen können sowie eine gute elektrostatische Abschirmung über die gesamte Fläche hinweg sicher gewährleistet ist. Bei einer Ausführungsform

EP 0 065 316 B2

hat sich beispielsweise gezeigt, daß durch die erfindungsgemäßen Maßnahmen elektrostatische Felder mit 20 000 Volt und darüber auf 2500 Volt und darunter begrenzt gehalten werden konnten. Dies ist sowohl hinsichtlich des Schlaf und Wohlgefühls als auch hisichtlich des Schlafverhaltens für den Schläfer sehr vorteilhaft Dieselben Vorteile werden auch bei einem Betteil mit einer Einlage mit Kupferelementen erreicht, wenn die Einlage eine kupferbedampfte Folie ist.

Gemäß einer bevorzugten Ausführungsform der Erfindung sind die Kupferlitzen in Abständen von 2 bis 20, vorzugsweise von 5 bis 15 und insbesondere von 9 bis 11 mm in das Gewebe eingewebt. Die nebeneinanderliegenden Kupferlitzen können auch untereinander verbunden oder eingewebt sein.

Vorteilhaft ist es, wenn die Einlagen einen Widerstand von 0,1 bis 100, vorzugsweise von 1 bis 20 und insbesondere von 10 bis 11 Ohm/cm aufweisen. Bei diesen Widerstandswerten ergaben sich sehr gute antistatische Effekte im Bettbereich.

Die Einlage sollte wenigstens auf einer Seite einer Betteinlage vorgesehen sein. Es ist jedoch auch möglich, im Ober- oder Unterbett auch mehrere parallel zueinander liegende Einlagen vorzusehen, wenn dies für eine besonders gute Abschirmung des elektrostatischen Feldes erforderlich sein sollte. Die einlage kann vorzugsweise auf der Innenseite eines Überzugs oder im Oberbett vorgesehen sein.

Gemäß einer weiteren bevorzugten Ausführungsform der Erfindung ist zusätzlich zur Einlage eine wärmerückstrahlende Schicht vorgesehen, die Wärme ins Innere des Bettes zurückstrahlt, so daß Betteil, beispielsweise das Oberbett, leichter und mit weniger Material dieselbe Wärmewirkung erzielt. Die wärmerückstrahlende Schicht kann dabei vorzugsweise aus einem mit Aluminium behandelten oder be dampften Gewebe bestehen.

Es sei noch darauf hingewiesen, daß die erfindungsgemäße Einlage nicht nur hinsichtlich des elektrischen Feldes, sondern auch hinsichtlich eines Ionenaustauschs vorteilhafte physikalische Eigenschaften für den Bettbereich aufweist, wodurch ebenfalls das Wohlgefühl der schlafenden Person deutlich verbessert wird und sich ein tieferer und gesünderer Schlaf ergibt.

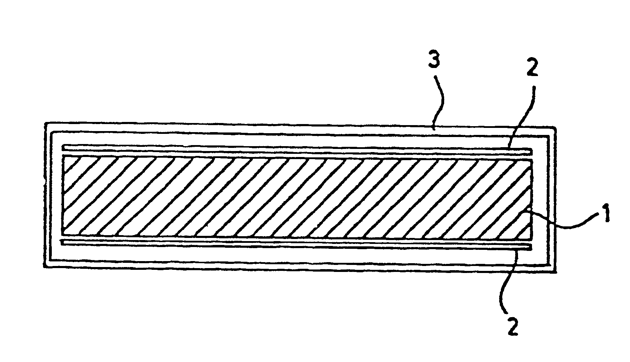

Die Erfindung wird nachfolgend anhand der einzigen Zeichnung beispielsweise näher erläutert. Die Zeichnung zeigt ein Unterbett im Querschnitt. Auf eine Schaumstoff- oder sonstige Einlage 1 ist wenigstens auf einer Seite derselben eine Kupfer- bzw. Kupfersulfideinlage 2 in Form eines entsprechend behandelten Gewebes bzw. in Form eines Gewebes mit eingewebten Kupferlitzen aufgeklebt, aufgesteppt, aufkaschiert oder in einer sonstigen Weise befestigt. Um die aus der Schaumstoffschicht 1 und den beiden Kupfer- bzw. Kupfersulfideinlagen bestehende gesamte Unterbetteinlage herum befindet sich der Unterbettuberzug 3. Selbstverständlich ist es auch möglich, die Kupfer- bzw. Kupfersulfideinlage auch am Bettüberzug 3 anzubringen.

Wie bereits zuvor anhand von Ausführungs beispielen und bevorzugten Ausführungsformen dergelegt, sind verschiedene Abwandlungen des erfindungsgemäßen Ausführungsbeispiels je nach den Umständen. Erfordernissen und Wünschen möglich, ohne daß dadurch der Erfindungsgedanke verlassen wird. Insbesondere ist es möglich, die erfindungsgemäße Kupfer- bzw. Kupfersulfideinlage auch in Oberbetten, Kissen oder sonstigen Decken oder Schlafdecken vorzusehen. Es ist weiterhin möglich durch Kombination einer wärmerückstrahlenden Schicht bzw. -einlage mit einer Kupfer- bzw. Kupfersulfideinlage die Vorteile beider Schichten bzw. Einlagen in geeignneter Weise zu kombinieren und dadurch Betteile zu schaffen, die ein optimales physiologisches Wohlgefühl beim Schlafen eintreten lassen und zu einem tieferen, gesünderen und längeren Schlaf führen.

## Patentansprüche

1. Betteil, insbesondere Ober- oder Unterbett, das ein Gewebeeinlage (2) mit Kupferelementen aufweist, dadurch gekennzeichnet, daß die Kupferelemente in Form von Kupferlitzen in die Gewebeeinlage (2) eingewebt sind.

2. Betteil, insbesondere Ober- oder Unterbett, das eine Gewebeeinlage (2) mit Kupferelementen aufweist, dadurch gekennzeichnet, daß die Kupferelemente aus in Form von auf die Gewebeeinlage (2) aufgedampftem Kupfer besteht.

3. Betteil, insbesondere Ober- oder Unterbett, das eine Einlage (2) mit Kupferelementen aufweist, dadurch gekennzeichnet, daß die Einlage (2) eine mit Kupfer bedampfte Folie ist.

4. Betteil nach Anspruch 1, dadurch gekennzeichnet, daß die Kupferlitzen in Abständen von 2 bis 20, vorzugsweise von 5 bis 15 und insbesondere von 9 bis 11 mm in das Gewebe eingewebt sind.

5. Betteil nach einem der Ansprüche 1 bis 4, dadurch gekennzeichnet, daß die Einlage (2) einen Widerstand von 0,1 bis 100, vorzugsweise von 1 bis 20 und insbesondere von 10 bis 11 Ohm/cm aufweist.

6. Betteil nach einem der Ansprüche 1 bis 4, dadurch gekennzeichnet, daß die Einlage (2) wenigstens auf einer Seite einer Betteinlage (1) vorgesehen ist.

3

7. Betteil nach einem der Ansprüche 1 bis 4, dadurch gekennzeichnet, daß die Einlage (2) auf der Innenseite eines Überzugs (3) vorgesehen ist.

8. Betteil nach einem der Ansprüche 1 bis 4, dadurch gekennzeichnet, daß die Einlage (2) im Oberbett vorgesehen ist.

9. Betteil nach einem der Ansprüche 1 to 8, dadurch gekennzeichnet, daß zusätzlich zur Einlage (2) eine wärmerückstrahlende Schicht vorgesehen ist.

10. Betteil nach Anspruch 9, dadurch gekennzeichnet, daß die mit Kupferelementen versehenen Bereiche mit Bereichen der wärmerückstrahlenden Schicht abwechseln.

11. Betteil nach Anspruch 9 und 10, dadurch gekennzeichnet, daß die wärmerückstrahlende Schicht ein mit Aluminium bedampftes Gewebe ist.

## Claims

1. A bed part, particularly an upper or a lower bed, which comprises a fabric inlay (2) with copper elements, characterised in that the copper elements are in the form of copper strands and are woven into the fabric inlay (2).

2. A bed part, particularly an upper or a lower bed, comprising a fabric inlay (2) with copper elements, characterised in that the copper element comprises copper applied to the fabric inlay (2) by vapour deposition.

3. A bed part, particularly an upper or lower bed, comprising an inlay (2) with copper elements, characterised in that the inlay (2) is a foil having vapour deposited copper.

4. A bed part as claimed in Claim 1, characterised in that the copper strands are woven into the fabric so as to be spaced apart by distances of from 2 to 20, preferably from 5 to 15, and particularly from 9 to 11 mm.

5. A bed part as claimed in one of the claims 1 to 4, characterised in that the inlay (2) has resistance of from 0.1 to 100, preferably of from 1 to 20, and particularly from 10 to 11 ohm/cm.

6. A bed part as claimed in one of the claims 1 to 4, characterised in that the inlay (2) is provided at least on one side of a bed inlay (1).

7. A bed part as claimed in one of the claims 1 to 4, characterised in that the inlay (2) is provided on the inside of a cover (3).

8. A bed part as claimed in one of the claims 1 to 4, characterised in that the inlay (2) is provided in the upper bed.

9. A bed part as claimed in one of the claims 1 to 8, characterised in that a heat-reflecting layer is provided in addition to the inlay (2).

10. A bed part as claimed in claim 9, characterised in that the regions provided with copper elements alternate with regions of the heat-reflecting layer.

11. A bed part as claimed in claims 9 and 10, characterised in that the heat-reflecting layer is a fabric to which aluminium is applied by vapour deposition.

## Revendications

1. Elément de lit, notamment couvre-lit ou des sous de lit, qui présente une insertion de tissu (2) munie d'éléments en cuivre, caractérisé en ce que les éléments en cuivre sont incorporés par tissage dans l'insertion en tissu (2) sous la forme de tresses de cuivre.

2. Elément de lit, notamment couvre lit ou dessous de lit, qui présente une insertion en tissu (2) munie d'éléments en cuivre, caractérisé en ce que les éléments en cuivre sont réalisés sous la forme de cuivre déposé par évaporation sur l'insertion en tissu (2).

3. Elément de lit, notamment couvre lit ou dessous de lit, qui présente une insertion (2) munie d'éléments en cuivre, caractérisé en ce que l'insertion (2) est une pellicule munie d'un revêtement du cuivre déposé par évaporation.

4. Elément de lit selon la revendication 1, caractérisé, en ce que les tresses de cuivre sont incorporées par tissage dans le tissu à des espacements de 2 à 20, de préférence 1 à 15 et en particulier de 9 à 11 mm.

5. Elément de lit selon l'une des revendications 1 à 4, caractérisé en ce que l'insertion (2) présente une resistance de 0,01 à 100, de préférence de 1 à 20 et en particulier de 10 à 11 ohms/cm.

6. Elément de lit selon l'une des revendications 1 à 4, caractérisé en ce que l'insertion (2) est prévue au moins sur une face d'une garniture de lit (1).

7. Elément de lit selon l'une des revendications 1 à 4, caractérisé en ce que l'insertion (2) est prévue sur la face interne d'un revêtement (3).

8. Elément de lit selon l'une des revendications 1 à 4, caractérisé en ce que l'insertion (4) est prévue dans le couvre-lit.

9. Elément de lit selon l'une des revendications 1 à 8, caractérisé en ce qu'il est prévu, en supplément de l'insertion (2), une couche réfléchissante thermique.

10. Elément de lit selon la revendication 9, caractérisé en ce que les régions munies d'élements en cuivre alternent avec des régions de la couche réfléchissante thermique.

11. Elément de lit selon les revendications 9 et 10, caractérisé en ce que la couche réfléchissante thermique est un tissu muni d'aluminium déposé par évaporation.